(19) Europäisches Patentamt
European Patent Office
Office européen des brevets

(11) Veröffentlichungsnummer : **0 182 168 B1**

(12) **EUROPÄISCHE PATENTSCHRIFT**

(45) Veröffentlichungstag der Patentschrift : **27.07.88**

(51) Int. Cl.⁴ : **C 07 C 67/343**, C 07 C 69/734

(21) Anmeldenummer : **85113943.6**

(22) Anmeldetag : **02.11.85**

(54) **Verfahren zur Herstellung von Hydroxymethylen-alkoxyessigsäureestern.**

(30) Priorität : **13.11.84 DE 3441369**

(43) Veröffentlichungstag der Anmeldung : **28.05.86 Patentblatt 86/22**

(45) Bekanntmachung des Hinweises auf die Patenterteilung : **27.07.88 Patentblatt 88/30**

(84) Benannte Vertragsstaaten : **BE CH DE FR GB IT LI NL**

(56) Entgegenhaltungen : **EP-A- 0 159 599**

(73) Patentinhaber : **BAYER AG**
**Konzernverwaltung RP Patentabteilung**
**D-5090 Leverkusen 1 Bayerwerk (DE)**

(72) Erfinder : **Arndt, Michael, Dr.**
**Cäcilienstrasse 5**
**D-5600 Wuppertal 1 (DE)**
Erfinder : **Maurer, Fritz, Dr.**
**Roeberstrasse 8**
**D-5600 Wuppertal 1 (DE)**

**EP 0 182 168 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein neues Verfahren zur Herstellung von Hydroxymethylen-alkoxyessigsäureestern bzw. ihren Alkalisalzen aus Ameisensäureester und Alkoxyessigsäureester in Gegenwart einer alkoholischen Alkoholatlösung.

Es ist bereits bekannt, daß Hydroxymethylenverbindungen erhalten werden, wenn man Ameisensäureester und Carbonsäureester in Gegenwart eines inerten Verdünnungsmittels, wie z. B. Diethylether und in Gegenwart von alkoholfreien Alkalialkoholaten umsetzt. Die Nachteile bei diesem Verfahren bestehen darin, daß bei der Herstellung größerer Mengen an Hydroxymethylen-Verbindungen die Viskosität der Reaktionsmischung während der Reaktion so zunimmt, daß dadurch die Rührbarkeit und die Wärmeabfuhr wesentlich beeinträchtigt werden und die Ausbeuten unbefriedigend sind (vgl. Houben-Weyl-Müller, Band VII/1, S. 46-49, Thieme-Verlag Stuttgart). Außerdem ist bekannt, daß derartige Esterkondensationen unter Kohlenoxiddruck zu besseren Ergebnissen führen. Nachteilig bei diesem Verfahren sind die technisch und sicherheitstechnisch aufwendige Reaktionsdurchführung, Kohlenmonoxid wird bei diesem Verfahren unter einem Druck von 30 bis 40 atm eingesetzt (vgl. Houben-Weyl-Müller, Band VII/1, S. 46-49, Thieme-Verlag Stuttgart).

Weiterhin ist allgemein bekannt, daß es sich bei allen Teilreaktionen der Esterkondensation um Gleichgewichtsreaktionen handelt und eine hohe Ausbeute nur dann gewährleistet ist, wenn die Reaktion in Gegenwart von alkoholfreiem Alkoholat durchgeführt wird.

Es wurde nun gefunden, daß man Hydroxymethylen-alkoxyessigsäureester der Formel I

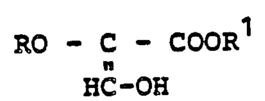

(I)

in welcher R und $R^1$ gleich oder verschieden sind und für Alkyl mit 1 bis 4 Kohlenstoffatomen stehen, bzw. ihre Alkalisalze erhält, wenn man eine Mischung aus einem inerten Verdünnungsmittel und einer alkoholischen Alkalialkoholat-Lösung herstellt, aus dieser Mischung 60 bis 80 Gew.-% Alkohol destillativ entfernt und anschließend mit einem Gemisch aus Alkoxyessigsäureester der Formel II

$$RO—CH_2COOR^1 \qquad (II)$$

in welcher R und $R^1$ die oben angegebenen Bedeutungen haben, und Ameisensäureester der Formel III

$$HCOOR^2 \qquad (III)$$

in welcher $R^2$ für $C_1$-$C_4$-Alkyl steht, bei einer Temperatur von 0 °C bis 40 °C umsetzt.

Überraschenderweise ist es möglich mit Hilfe des erfindungsgemäßen Verfahrens eine Esterkondensation, in Gegenwart von preisgünstigeren alkoholischen Alkoholatlösungen, in sehr hohen Ausbeuten durchzuführen. Weitere Vorteile des erfindungsgemäßen Verfahrens bestehen darin, daß durch die Verwendung von alkoholischen Alkoholatlösungen keine wesentliche Steigerung der Viskosität stattfindet, dadurch eine problemlose Reaktionwärmeabfuhr gewährleistet ist und der Einsatz von sicherheitstechnisch aufwendigen Druckapparaturen nicht notwendig ist, sondern Apparaturen eingesetzt werden können, die in großtechnischen Anlagen üblich sind.

Bevorzugt werden mit dem erfindungsgemäßen Verfahren die Verbindungen der Formel (I) hergestellt, in welcher R und $R^1$ gleich oder verschieden sind und für Alkyl mit 1 bis 4 Kohlenstoffatomen stehen. Besonders bevorzugt werden mit dem erfindungsgemäßen Verfahren die Verbindungen der Formel (I) hergestellt, in welcher R und $R^1$ gleich oder verschieden sind und für Methyl, Ethyl oder n-Propyl stehen.

Als Alkalisalze der Verbindungen der Formel (I) kommen bevorzugt bzw. insbesondere bevorzugt die Natriumsalze in Frage.

Verwendet man für das erfindungsgemäße Verfahren Methoxyessigsäuremethylester, Ameisensäuremethylester und eine methanolische Natriummethanolat-Lösung als Ausgangsstoffe, so kann die Reaktion durch das folgende Formelschema skizziert werden :

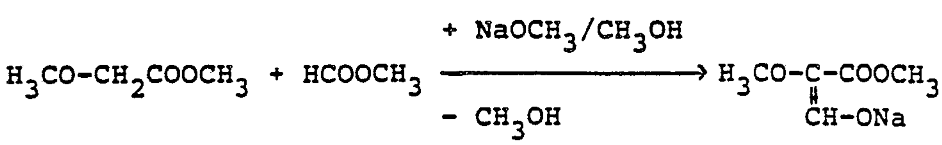


Die zur Durchführung des erfindungsgemäßen Verfahrens als Ausgangsstoffe benötigten Alkoxyessigester sind durch die Formel (II) allgemein definiert. In dieser Formel haben R und $R^1$ vorzugsweise diejenigen Bedeutungen, welche oben bei der Definition der Formel (I) angegeben sind.

Als Beispiele für die Verbindungen der Formel (II) seien genannt :

Methoxy-, Ethoxy-, n-Propoxy-essigsäuremethylester, -essigsäureethylester und -essigsäure-n-propylester.

Die weiterhin für das erfindungsgemäße Verfahren zu verwendenden Ameisensäureester sind durch die Formel (III) allgemein definiert. In dieser Formel steht $R^2$ bevorzugt für $C_1$-$C_2$-Alkyl.

Als Beispiele für die Verbindungen der Formel (III) seien genannt :

Ameisensäuremethylester und Ameisensäureethylester.

Die Verbindungen der Formel (II) und (III) sind allgemein bekannte Verbindungen der organischen Chemie.

Das erfindungsgemäße Verfahren zur Herstellung der Verbindungen der Formel (I) wird unter Verwendung von inerten Verdünnungsmitteln durchgeführt. Hierzu gehören insbesondere aromatische Kohlenwasserstoffe wie Benzol, Xylol, Toluol und Ether, wie Methyl-tert.-butylether, Diethyl- und Dibutylether. Besonders bevorzugt wird Xylol als Verdünnungsmittel eingesetzt.

Das erfindungsgemäße Verfahren wird in Gegenwart einer alkoholischen Alkoholatlösung durchgeführt. Besonders bewährt haben sich Alkalialkoholate, wie Natrium-methylat und -ethylat. Als Alkohol wird beim Einsetzen von Natrium-methylat, Methanol und beim Einsatz von Natriumethylat Ethanol eingesetzt.

Das erfindungsgemäße Verfahren wird im allgemeinen bei Temperaturen zwischen 0 °C und 40 °C durchgeführt. Bevorzugt wird der Bereich zwischen 0 °C und 20 °C. Die Umsetzungen werden bei Normaldruck durchgeführt.

Zur Durchführung des erfindungsgemäßen Verfahrens setzt man auf 1 Mol der Verbindung der Formel (II) 1 bis 3 Mol, vorzugsweise 1,8 bis 2,2 Mol Ameisensäureester der Formel (III) und 1 bis 2 Mol, vorzugsweise 1,3 bis 1,7 Mol Alkalialkoholat ein. Die alkoholische Alkalialkoholatlösung enthält auf 1 Mol Alkalialkoholat vor Destillation, 3,5 bis 5,5-Mol, vorzugsweise 4 bis 5 Mol Alkohol und nach der Destillation 0,6 bis 2,2 Mol, vorzugsweise 0,8 bis 2,0 Mol Alkohol. Die Aufarbeitung der Verbindungen der Formel (I) kann nach üblichen Methoden durchgeführt werden. Vorzugsweise wird das Reaktionsprodukt ohne weitere Aufarbeitung in die nächste Umsetzung eingesetzt.

Die nach dem erfindungsgemäßen Verfahren herstellbaren Verbindungen den Formel (I) bzw. ihre Alkalisalze sind wichtige Zwischenprodukte für die Herstellung von bekannten Schädlingsbekämpfungsmitteln (vgl. z. B. DE-OS 2 928 185).

Die Weiterverarbeitung der Verbindungen der Formel (I) zu bekannten Schädlingsbekämpfungsmittel sei beispielhaft anhand des folgenden Schemas verdeutlicht :

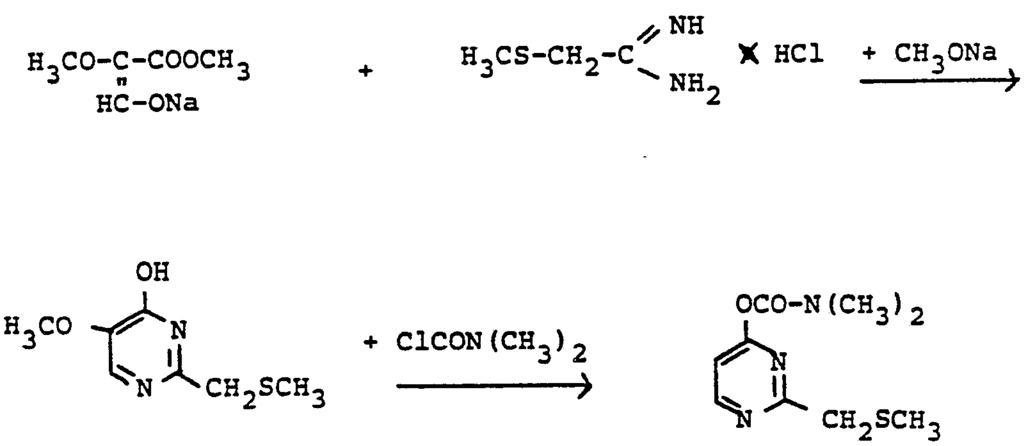


N,N-Dimethyl-O-pyrimidinyl-carbaminsäureester

Herstellungsbeispiel

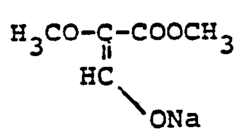

Aus einer Mischung aus 315 g 25 %ige methanolische Natriummethylatlösung und 500 ml Xylol (technisches. Isomerengemisch) werden 160 g Methanol abdestilliert. Dann werden bei 5 °C bis 10 °C

104 g Methoxyessigsäuremethylester und 120 g Methylformiat gleichzeitig zugegeben. Anschließend wird eine Stunde bei 20 °C nachgerührt, die Phasen getrennt und die Phase, die das Reaktionsprodukt enthält wird ohne weitere Aufarbeitung in die nachfolgende Reaktion eingesetzt.

Man erhält auf diese Weise 437 g einer 30 %igen (85 % der Theorie) Hydroxymethylen-methoxyessigsäuremethylester-Natriumsalz-Lösung.

Vergleichsbeispiel

In einem Druckreaktor werden 1 Mol Methoxyessigsäuremethylester und 2 Mol Methylformiat vorgelegt und bei 0 bis 10 °C 1 Mol festes Natriummethylat zudosiert. Der Reaktor wird geschlossen und bei 70 °C unter einem Kohlenmonoxiddruck von 40 atm so lange gerührt, bis kein Kohlenmonoxid mehr aufgenommen wird. Nach beendeter Reaktion wird filtriert, mit Methylformiat nachgewaschen und getrocknet.

Man erhält 131 g (85 % der Theorie) Hydroxymethylen-methoxyessigsäuremethylester-Natriumsalz.

**Patentansprüche**

1. Verfahren zur Herstellung von Hydroxymethylenalkoxyessigsäureester der Formel (I)

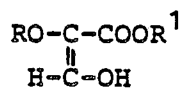

(I)

in welcher R und $R^1$ gleich oder verschieden sind und für Alkyl mit 1 bis 4 Kohlenstoffatomen stehen, bzw. von Alkalisalzen der vorgenannten Ester, dadurch gekennzeichnet, daß man eine Mischung aus einem inerten Verdünnungsmittel und einer alkoholischen Alkalialkoholat-Lösung herstellt, aus dieser Mischung 60 bis 80 Gewichtsprozent Alkohol destillativ entfernt und anschließend mit einem Gemisch aus Alkoxyessigester der Formel (II)

$$RO—CH_2COOR^1$$

in welcher R und $R^1$ die oben angegebene Bedeutung haben, und Ameisensäureester der Formel (III)

$$HCOOR^2 \qquad (III)$$

in welcher $R^2$ für $C_1$-$C_4$-Alkyl steht, bei einer Temperatur von 0 °C bis 40 °C umsetzt.

2. Verfahren gemäß Anspruch 1, dadurch gekennzeichnet, daß es sich bei den inerten Verdünnungsmittel um ein Verdünnungsmittel der Reihe aromatische Kohlenwasserstoffe oder Ether handelt.

3. Verfahren gemäß Anspruch 1 bis 2, dadurch gekennzeichnet, daß es sich bei dem inerten Verdünnungsmittel um ein Verdünnungsmittel der Reihe Benzol, Xylol, Toluol, Methyl-tert.-butylether, Diethylether und Dibutylether handelt.

4. Verfahren gemäß Anspruch 1 bis 3, dadurch gekennzeichnet, daß es sich beim Alkalialkoholat um Natriummethylat oder Natriumethylat handelt.

5. Verfahren gemäß Anspruch 1 bis 4, dadurch gekennzeichnet, daß es sich beim Alkohol um Methanol oder Ethanol handelt.

6. Verfahren gemäß Anspruch 1 bis 5, dadurch gekennzeichnet, daß man die Reaktion zwischen 0 und 20 °C durchführt.

7. Verfahren gemäß Anspruch 1 bis 6, dadurch gekennzeichnet, daß man pro Mol der Verbindung (II) 1 bis 3 Mol Ameisensäureester (III) und 1 bis 2 Mol Alkalialkoholat einsetzt.

**Claims**

1. Process for the preparation of a hydroxymethylenealkoxyacetic acid ester of the formula (I)

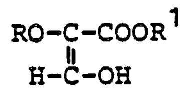

(I)

in which R and $R^1$ are identical or different and represent alkyl having 1 to 4 carbon atoms, and/or of alkali metal salts of the above mentioned esters, characterised in that a mixture of an inert diluent and an alcoholic solution of alkali metal alcoholate is prepared, 60 to 80 % by weight of the alcohol are removed from this mixture by distillation, and the residue is then reacted at a temperature of 0 °C to 40 °C with a mixture of an alkoxyacetic ester of the formula (II)

$$RO—CH_2—COOR^1$$

in which R and $R^1$ have the meaning indicated above, and a formic acid ester of the formula (III)

$$HCOOR^2 \qquad (III)$$

in which $R^2$ represents $C_1$-$C_4$-alkyl.

2. Process according to Claim 1, characterised in that the inert diluent is a diluent from the series comprising aromatic hydrocarbons or ethers.

3. Process according to Claim 1 to 2, characterised in that the inert diluent is a diluent from the series comprising benzene, xylene, toluene, methyl tert.-butyl ether, diethyl ether and dibutyl ether.

4. Process according to Claim 1 to 3, characterised in that the alkali metal alcoholate is sodium methylate or sodium ethylate.

5. Process according to Claim 1 to 4, characterised in that the alcohol is methanol or ethanol.

6. Process according to Claim 1 to 5, characterised in that the reaction is carried out between 0 and 20 °C.

7. Process according to Claim 1 to 6, characterised in that 1 to 3 moles of formic acid ester (III) and 1 to 2 moles of alkali metal alcoholate are employed per mole of the compound (II).

**Revendications**

1. Procédé de fabrication d'esters hydroxyméthylène-alcoxyacétiques de formule (I)

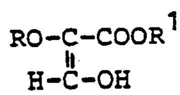

(I)

dans laquelle R et $R^1$ sont identiques ou différents et représentent un alcoyle ayant 1 à 4 atomes de carbone, ou des sels alcalins des esters précités, caractérisé en ce qu'on prépare un mélange d'un diluant inerte et d'une solution alcoolique d'un alcoolate alcalin, on élimine à partir de ce mélange 60 à 80 % en poids de l'alcool par distillation, puis on le fait réagir avec un mélange d'un ester alcoxyacétique de formule (II) :

$$RO—CH_2COOR^1 \qquad (II)$$

dans laquelle R et $R^1$ ont la signification indiquée plus haut, et d'un ester formique de formule (III)

$$HCOOR^2 \qquad (III)$$

dans laquelle $R^2$ représente un alcoyle en $C_1$ à $C_4$, à une température de 0 °C à 40 °C.

2. Procédé selon la revendication 1, caractérisé en ce que dans le cas des diluants inertes il s'agit d'un diluant de la série des hydrocarbures aromatiques ou des éthers.

3. Procédé selon les revendications 1 à 2, caractérisé en ce que dans le cas du diluant inerte il s'agit d'un diluant de la série du benzène, xylène, toluène, méthyl-t-butyl-éther, diéthyl-éther et dibutyl-éther.

4. Procédé selon les revendications 1 à 3, caractérisé en ce que dans le cas de l'alcoolate alcalin il s'agit de méthylate de sodium ou d'éthylate de sodium.

5. Procédé selon les revendications 1 à 4, caractérisé en ce que dans le cas de l'alcool il s'agit de méthanol ou d'éthanol.

6. Procédé selon les revendications 1 à 5, caractérisé en ce qu'on exécute la réaction entre 0 et 20 °C.

7. Procédé selon les revendications 1 à 6, caractérisé en ce que par mole du composé (II) on utilise 1 à 3 moles d'ester formique (III) et 1 à 2 moles d'alcoolate alcalin.